# EUROPEAN PATENT APPLICATION

(11) **EP 4 278 895 A1**
(43) Date of publication of application: **22.11.2023**
(21) Application number: 22173556.6
(22) Date of filing: 16.05.2022
(51) Int. Cl.: A01N 1/02, C12N 1/20, C09K 3/24

(54) **ICE-NUCLEATING COMPOSITION**

(71) Applicant: Max-Planck-Gesellschaft zur Förderung der Wissenschaften e.V., 80539 München (DE)
(72) Inventor: MEISTER, Konrad, 55128 Mainz (DE); SCHWIDETZKY, Ralph, 55128 Mainz (DE); BONN, Mischa, 55128 Mainz (DE)
(74) Representative: Maiwald GmbH

(57) **Abstract**

The present invention relates to an aqueous composition which has a pH of from 5 to 8 and comprises (i) 0.005 mg/ml or less of bacteria, bacterial ghosts or fragments thereof which carry an ice nucleation protein, (ii) a buffer, and (iii) a polyfunctional compound comprising two or more functional groups, wherein the functional groups are selected from a hydroxy group, an ether group, a carboxylic acid or carboxylate group, a carboxylic acid ester group, an amine group and an amide group.

## Description

The present invention relates to a composition which promotes ice nucleation in supercooled water.

The formation of ice from water is thermodynamically favored at temperatures below 0 °C, but the crystallization process is kinetically hindered. As a result, pure water can be supercooled to temperatures as low as -38 °C, below which homogenous ice nucleation occurs.

Ice nucleation at higher temperatures requires the presence of heterogeneous ice nucleation agents (INAs). INAs are ubiquitous in the environment and can be of abiotic and biological origin.

Ice-nucleating bacteria like *Pseudomonas syringae* are among the most effective ice nucleators known and affect freezing processes in agriculture, the atmosphere, and the biosphere. The ability of ice-nucleating bacteria to facilitate ice formation is attributed to specific proteins (ice nucleation proteins INPs) anchored to the outer bacterial cell membrane.

Snomax^{®} is a commercial product made of nonviable cells and cell fragments of *Pseudomonas syringae* (which is a bacterium carrying an ice nucleation protein) and is used for snowmaking.

Reviews about bacterial ice nucleation and the structure of ice nucleation proteins INPs (e.g. the amino acid sequences of these INPs) are provided by the following publications:
- P.K. Wolber, "Bacterial Ice Nucleation", Advances in Microbial Physiology, Vol. 34, 1993, pp. 203-237;
- J.S.H. Lorv et al., "Bacterial Ice Crystal Controlling Proteins", Scientifica, Volume 2014, Article ID 976895;
- K. Meister et al., "Toward Understanding Bacterial Ice Nucleation", J. Phys. Chem. B, 2022, 126, pp. 1861-1867.

Further information on ice nucleation proteins (e.g. amino acid sequences, bacteria containing these INPs) is provided by databases such as UniProt.

R. Schwidetzky et al., Chemistry - A European Journal, 2021, Vol. 27, Issue 26, pp. 7402-7407, describe the effect of ions on the efficiency of ice nucleating bacteria.

WO 2021/063943 A1 discloses a bacterial preparation comprising inactivated bacteria, bacterial ghosts or fragments thereof which carry an ice nucleation protein and which are fixed with a fixation agent.

WO 2018/005802 A1 discloses a hydrogel particle comprising an ice nucleating agent such as Snomax^{®}.

W. Lubitz et al., Bioengineered, 2017, Vol. 8, No. 5, pp. 488-500, study ice formation induced by bacterial ghosts (BGs) carrying an ice nucleation protein in their outer membrane.

Ice-nucleating bacteria (i.e. bacteria containing ice nucleation proteins) and their potential technical applications in artificial snowmaking and food processing are reviewed in the following publications:
- S.E. Lindow et al., "Bacterial ice nucleation: Significance and molecular basis", FASEB J.; 7, 1993, pp. 1338-1343;
- C.E. Morris et al., "Ice nucleation active bacteria and their potential role in precipitation", J. Phys. IV France, 121, 2004, pp. 87-103;

Biological ice-nucleating particles (such as ice-nucleating bacteria of the genera *Pseudomonas*, *Pantoea* and *Xanthomonas*) being present in the atmosphere are reviewed by W. Hu et al., "Overview of biological ice nucleating particles in the atmosphere", Environment International, 146, 2021, 106197.

Some low molecular polyhydroxy compounds such as glycerol, ethylene glycol and sugars, as well as some water soluble polymers such as polyethylene glycol and polyvinyl pyrrolidone are known to be effective cryoprotectants which suppress ice crystallization, see e.g. G. Zhao et al., "Ice Inhibition for Cryopreservation", Adv. Sci., 8, 2021, 2002425.

B. Jin et al., "Intracellular ice formation in mouse zygotes and early morulae vs. cooling rate and temperature-experimental vs. theory", Cryobiology, 73, 2016, pp. 181-186, and P. Mazur et al., "Extra- and intracellular ice formation in mouse oocytes", Cryobiology, 51, 2005, pp. 29-53, describe the freezing of oocytes and zygotes in compositions comprising Snomax^{®} (i.e. non-viable *Pseudomonas syringae* bacteria or fragments thereof), a phosphate buffer, and ethylene glycol or glycerol. While Snomax^{®} has been added to minimize the degree of supercooling in the surrounding aqueous medium (i.e. thereby promoting extracellular ice nucleation), ethylene glycol or glycerol has been added as a cryoprotective agent to suppress intracellular ice nucleation. The ice-nucleating bacteria are present at a concentration of 0,001 wt% (i.e. 0,01 mg/ml).

The use of ice-nucleating bacteria for food processing, snowmaking or similar applications has raised concerns about harmful effects on health or the environment, even if non-viable bacterial cells are used. However, if the concentration of the ice-nucleating bacteria is reduced (thereby minimizing the risk of any harmful side effects), this will adversely affect ice nucleation properties. Typically, the higher the concentration of the ice-nucleating bacteria in the aqueous medium, the less supercooling (and therefore the less energy) is needed for freezing the aqueous medium.

An object of the present invention is to provide a composition which comprises ice-nucleating particles and is still a very effective ice nucleator even if the concentration of the ice-nucleating particles is rather low.

The object is solved by an aqueous composition comprising
(i) 0.005 mg/ml or less of bacteria, bacterial ghosts or fragments thereof which carry an ice nucleation protein,
(ii) a buffer,
(iii) a polyfunctional compound comprising two or more functional groups, wherein the functional groups are selected from a hydroxy group, an ether group, a carboxylic acid or carboxylate group, a carboxylic acid ester group, an amine group and an amide group,
wherein the aqueous composition has a pH of from 5 to 8.

As the bacteria or bacterial ghosts or fragments of the bacteria or bacterial ghosts carry an ice nucleation protein, they represent ice nucleating particles. Typically, if the concentration of ice nucleating bacteria or bacterial ghosts or fragments thereof being present in an aqueous medium is reduced, ice nucleation activity (expressed by the temperature T₅₀ at which 50% of the water droplets are frozen) is reduced as well. However, the present invention has surprisingly found that the decrease of ice nucleation activity at decreasing concentration of ice-nucleating particles is significantly less if the aqueous medium additionally comprises a buffer for pH adjustment (pH of 5 to 8) and a polyfunctional compound (i.e. a compound comprising two or more functional groups selected from a hydroxy group, an ether group, a carboxylic acid or carboxylate group, a carboxylic acid ester group, an amine group and an amide group).

Accordingly, although the aqueous composition of the present invention comprises the ice-nucleating particles at a very low concentration (0.005 mg/ml or less), it still has a surprisingly high ice nucleation activity due to the presence of the buffer (adjusting the pH of the aqueous composition to a value of 5 to 8) and the polyfunctional compound.

Ice nucleation proteins (INPs), i.e. proteins which promote the nucleation of ice in supercooled water, are known to the skilled person. The same applies to bacteria, bacterial ghosts or fragments of bacteria or bacterial ghosts which carry an ice nucleation protein.

Information about the structure of ice nucleation proteins INPs (e.g. amino acid sequences of INPs) is provided by the following publications:
- P.K. Wolber, "Bacterial Ice Nucleation", Advances in Microbial Physiology, Vol. 34, 1993, pp. 203-237;
- J.S.H. Lorv et al., "Bacterial Ice Crystal Controlling Proteins", Scientifica, Volume 2014, Article ID 976895.

Exemplary ice nucleating proteins are InaZ, InaX, InaA, InaV, InaU, InaK, InaW and IceE.

Further information on ice nucleation proteins (INPs) such as InaZ, InaX, InaA, InaV, InaU, InaK, InaW and IceE (e.g. amino acid sequence, bacteria containing these INPs) is provided by databases such as UniProt Knowledgebase (UniProtKB).

**Table 1: Accession numbers of the ice nucleation proteins (INPs) InaZ, InaX, InaA, InaV, InaU, InaK, InaW and IceE in the database UniProt Knowledgebase (UniProtKB)**

| Name of INP | UniProtKB Accession Number |
|---|---|
| InaZ | P06620 (Entry version 94, 23 February 2022) |
| InaX | P18127 (Entry version 75, 23 February 2022) |
| InaA | P20469 (Entry version 70, 23 February 2022) |
| InaV | 033479 (Entry version 58, 2 June 2021) |
| InaU | Q47879 (Entry version 65, 23 February 2022) |
| InaK | 030611 (Entry version 60, 2 June 2021) |
| InaW | P09815 (Entry version 71, 23 February 2022) |
| IceE | P16239 (Entry version 72, 2 June 2021) |

Exemplary bacteria carrying an ice nucleation protein are *Pseudomonas syringae, Pseudomonas fluorescens, Xanthomonas campestris, Pantoea ananas, Pantoea agglomerans* and *Enterobacter agglomerans.* These are bacteria comprising internally produced ice nucleation proteins. However, it is also possible to provide or synthesize the ice nucleation protein externally, followed by fixing the ice nucleation protein to a membrane of bacteria, bacterial ghosts or fragments of bacteria or bacterial ghosts.

It might be preferred that the bacteria are inactivated bacteria. As commonly known, inactivated bacteria are bacteria which are no longer capable of reproduction. Preparation of inactivated bacteria may include heat treatment, irradiation, treatment with an inactivating agent and/or freeze drying. These microbial inactivation methods are known to the skilled person.

Fragments of bacteria (e.g. fragments of *Pseudomonas syringae*, *Pseudomonas fluorescens*, *Xanthomonas campestris*, *Pantoea ananas*, *Pantoea agglomerans* and *Enterobacter agglomerans*) can be obtained by methods commonly know to the skilled person, e.g. by an atomizer.

Bacterial Ghosts (BGs) are empty, non-living particles, which can be generated from bacteria through a process whereby the cell content is ejected through a hole formed in the cell envelope, thereby resulting in an empty cell envelope.

Fragments of bacterial ghosts can be obtained by methods commonly know to the skilled person, e.g. by an atomizer.

In a preferred embodiment, the bacteria are *Pseudomonas syringae.*

Preferably, the ice nucleation protein is InaZ. As already outlined above, the amino acid sequence of the ice nucleation protein InaZ is known, see e.g. the database UniProtKB (accession number: P06620, entry version 94, 23 February 2022), Fig. 14 of WO 2018/005802 A1, and Fig. 10A of WO 2021/063943 A1.

The bacteria, bacterial ghosts or fragments of the bacteria or bacterial ghosts are present in the aqueous composition at a concentration of 0.005 mg/ml (5 × 10⁻³ mg/ml) or less, more preferably 0.0005 mg/ml (5 × 10⁻⁴ mg/ml) or less.

Preferably, the concentration of the bacteria, bacterial ghosts or fragments of the bacteria or bacterial ghosts in the aqueous composition is within the range of 5 × 10⁻³ mg/ml to 5 × 10⁻⁷ mg/ml, more preferably 5 × 10⁻⁴ mg/ml to 5 × 10⁻⁶ mg/ml.

The aqueous composition of the present invention comprises a buffer and has a pH of from 5 to 8.

Preferably, the pH value of the aqueous composition is within the range of 6 to 8, more preferably 6.5 to 8.0.

Appropriate buffers by which the pH of the aqueous composition can be adjusted to a value of 5 to 8, preferably 6 to 8, more preferably 6.5 to 8.0, are commonly known to the skilled person.

The buffer might be a phosphate buffer, a zwitterionic buffer, or an alkanol amine buffer.

Typically, a phosphate buffer comprises H₂PO4⁻ or HPO4²⁻ or any mixture thereof.

A zwitterionic buffer is a buffer comprising at least one zwitterionic compound, i.e. a compound comprising both an acid group (e.g. a sulfonic acid group or a carboxylic acid group) and a basic group (e.g. an amine group). Exemplary zwitterionic buffers are those commonly known under the term "Good's buffer".

A preferred zwitterionic compound may comprise both a sulfonic acid group and an amine group (i.e. a sulfonic acid group and an amine group within the same molecule). The zwitterionic buffer can be a buffer comprising 3-(*N*-morpholino)propanesulfonic acid or a salt thereof (commonly known as "MOPS"), or a buffer comprising 4-(2-hydroxyethyl)-1-piperazineethanesulfonic acid or a salt thereof (commonly known as "HEPES").

The alkanol amine buffer might be a buffer comprising bis-tris methane, a buffer comprising bis-tris propane, or a buffer comprising triethanol amine.

Preferably, the buffer is a phosphate buffer which comprises H₂PO4⁻ or HPO4²⁻ or any mixture thereof.

Phosphate buffers such as PBS (Phosphate Buffered Saline) and DPBS (Dulbecco's Phosphate Buffered Saline), zwitterionic buffers (such as MOPS and HEMES) and alkanol amine buffers are commercially available.

Optionally, the aqueous composition may comprise one or more alkali metal salts (such as one or more alkali metal halides or sulfates, e.g. NaCl and/or KCl), one or more earth alkali metal salts (such as one or more earth alkali metal halides or sulfates, e.g. CaCl2 and/or MgCl₂), one or more aluminium salts (such as aluminium chloride or aluminium sulfate), one or more iron salts (such as iron chloride), or one or more manganese salts (such as manganese chloride).

According to an exemplary embodiment, the aqueous composition comprises one or more alkali metal salts (such as one or more alkali metal halides or sulfates, e.g. NaCl and/or KCl) and/or one or more earth alkali metal salts (such as one or more earth alkali metal halides or sulfates, e.g. CaCl₂ and/or MgCl₂).

The aqueous composition may comprise the above-mentioned one or more salts at a concentration of 0.001 mol/I to 2 mol/l, more preferably 0.01 mol/I to 1 mol/I. If two or more of the above-mentioned salts are present, the concentration represents the total concentration.

The aqueous composition of the present invention comprises a polyfunctional compound comprising two or more functional groups, wherein the functional groups are selected from a hydroxy group, an ether group, a carboxylic acid or carboxylate group, a carboxylic acid ester group, an amine group and an amide group. The two or more functional groups might be the same (e.g. at least two hydroxy groups) or may differ from each other (e.g. at least one hydroxy group and at least one ether group).

Preferably, the two or more functional groups of the polyfunctional compound are selected from a hydroxy group, an ether group, a carboxylic acid or carboxylate group, and a carboxylic acid ester group. It might be preferred that the polyfunctional compound does not comprise an amine group and/or an amide group.

According to an exemplary embodiment, the polyfunctional compound may comprise at least two hydroxy groups and optionally one or more functional groups which are selected from an ether group, a carboxylic acid or carboxylate group and a carboxylic acid ester group (e.g. a carboxylic acid C₁₋₄ alkyl ester group).

The polyfunctional compound might be a monoalkylene glycol, a polyalkylene glycol (also known as polyalkylene oxide), glycerol, a polyvinyl alcohol or a polysaccharide.

The monoalkylene glycol might be ethylene glycol or propylene glycol.

The polyalkylene glycol might be a homo- or copolymer, e.g. an ethylene glycol homo- or copolymer or a propylene glycol homo- or copolymer. If the polyalkylene glycol is a copolymer, it might be a block polymer comprising at least one polyethylene glycol block and at least one polypropylene glycol block (e.g. a triblock polymer comprising a polypropylene glycol block which is flanked by two polyethylene glycol blocks). Such polyethylene glycol polypropylene glycol block polymers are commercially available (e.g. under the trade name Pluronic^{®}).

The polyvinyl alcohol can be a homopolymer or a copolymer (e.g. comprising vinyl acetate comonomer units).

The polysaccharide might be alginic acid or a salt thereof (i.e. an alginate).

According to another exemplary embodiment, the polyfunctional compound may comprise just one hydroxy group and at least one ether group.

The polyfunctional compound might be a monoalkylene glycol ether (e.g. a monoethylene glycol mono-C₁₋₄-alkyl ether or a monopropylene glycol mono-C₁₋₄-alkyl ether), a polyalkylene glycol ether (e.g. a polyethylene glycol mono-C₁₋₄-alkyl ether or a polypropylene glycol mono-C₁₋₄-alkyl ether).

If the polyfunctional compound is a polymer, its number average molecular weight might be within the range of 200 g/mol to 500,000 g/mol. However, polymers having an average molecular weight above or below said range might be used as well.

It might be preferred that the polyfunctional compound is not a zwitterionic compound i.e. a compound comprising both an acid group (e.g. a sulfonic acid group or a carboxylic acid group) and a basic group (e.g. an amine group). It might also be preferred that the polyfunctional compound is not a compound which comprises both at least one hydroxy group and at least one amine group.

The aqueous composition may comprise the polyfunctional compound at a concentration of 40 wt% or less, more preferably 15 wt% or less, e.g. 0.01 wt% to 40 wt%, more preferably 0.1 wt% to 15 wt%.

As already outlined above, the present invention has surprisingly found that the decrease of ice nucleation activity at decreasing concentration of ice-nucleating particles is significantly less if the aqueous medium additionally comprises a buffer for pH adjustment (pH of 5 to 8) and a polyfunctional compound (i.e. a compound comprising two or more functional groups selected from a hydroxy group, an ether group, a carboxylic acid or carboxylate group, a carboxylic acid ester group, an amine group and an amide group). Although the aqueous composition of the present invention comprises the ice-nucleating particles in a very low concentration (0.005 mg/ml or less), it still has a surprisingly high ice nucleation activity due to the presence of the phosphate buffer and the polyfunctional compound.

Accordingly, the present invention also relates to the use of the aqueous composition described above as an ice nucleating agent.

The aqueous composition of the present invention might be used for snowmaking (i.e. preparing artificial snow), freezing of foodstuff or foodstuff precursors, ex-vivo cryopreservation of biological samples (e.g. cells, organs) or cloud seeding.

The present invention also relates to the aqueous composition described above for use in ex-vivo cryopreservation of a biological sample (e.g. cells, an organ).

The present invention also relates to a process of forming artificial snow wherein the aqueous composition described above is sprayed into the air by a snowmaking machine (such as a snow gun or a snow lance). Cooling of the sprayed droplets results from water evaporation and/or the temperature of the air surrounding the water droplets.

The present invention also relates to a process of cloud seeding wherein the aqueous composition described above is sprayed in an atmospheric cloud (e.g. by a spraying device fixed to an aircraft).

The present invention also relates to a process of freezing a foodstuff or foodstuff precursor wherein the aqueous composition described above is in thermal contact with the foodstuff or foodstuff precursor and cooled to a temperature at which ice crystals are formed. The composition may contain the foodstuff or foodstuff precursor (i.e. direct contact between the aqueous composition and the foodstuff or foodstuff precursor).

The present invention also relates to a process of ex-vivo cryopreservation of a biological sample (e.g. cells, an organ, .... etc.) wherein the aqueous composition described above is in thermal contact with the biological sample outside of the human or animal body and is cooled to a temperature at which ice crystals are formed. The composition may contain the biological sample (i.e. direct contact between the aqueous composition and the biological sample).

### Examples

### Comparative Examples 1 and 2 (CE1 and CE2) and Inventive Examples IE1 to IE6

The aqueous composition of Comparative Example 1 (CE1) contained only bacteria and fragments thereof carrying an ice nucleation protein INP (in the following referred to as INP-carrying bacteria/bacterial fragments), but no buffer and no polyfunctional compound. The aqueous composition of CE1 had a pH of 6.2.

The aqueous composition of Comparative Example 2 (CE2) contained INP-carrying bacteria/bacterial fragments and a phosphate buffer (150 mM), but no polyfunctional compound. The aqueous composition of CE2 had a pH of 7.2.

The aqueous composition of Inventive Example 1 (IE1) contained INP-carrying bacteria/bacterial fragments, a phosphate buffer, and glycerol (1 wt%) as a polyfunctional compound. The aqueous composition of IE1 had a pH of 7.2.

The aqueous composition of Inventive Example 2 (IE2) contained INP-carrying bacteria/bacterial fragments, a phosphate buffer, and ethylene glycol (1 wt%) as a polyfunctional compound. The aqueous composition of IE2 had a pH of 7.2.

The aqueous composition of Inventive Example 3 (IE3) contained INP-carrying bacteria/bacterial fragments, a phosphate buffer, and polyethylene glycol (1 wt%, number average MW: 300 g/mol) as a polyfunctional compound. The aqueous composition of IE3 had a pH of 7.2.

The aqueous composition of Inventive Example 4 (IE4) contained INP-carrying bacteria/bacterial fragments, a phosphate buffer, and a polyethylene glycol polypropylene glycol block polymer (1 wt%, Pluronic^{®} F68) as a polyfunctional compound. The aqueous composition of IE4 had a pH of 7.2.

The aqueous composition of Inventive Example 5 (IE5) contained INP-carrying bacteria/bacterial fragments, a phosphate buffer, and polyvinyl alcohol (0,5 wt%, average MW: 11000 to 31000 g/mol) as a polyfunctional compound. The aqueous composition of IE5 had a pH of 7.2.

The aqueous composition of Inventive Example 6 (IE6) contained INP-carrying bacteria/bacterial fragments, a phosphate buffer, and sodium alginate (1 wt%) as a polyfunctional compound. The aqueous composition of IE6 had a pH of 7.2.

Snomax^{®} was used in CE1, CE2 and IE1 to IE6 as INP-carrying bacteria/bacterial fragments. As already mentioned above, Snomax^{®} is a commercial product made of cells and cell fragments of *Pseudomonas syringae* which is a bacterium carrying an ice nucleation protein.

The phosphate buffer used in CE2 and IE1 to IE6 was a commercially available PBS (phosphate-buffered saline) buffer.

Initially, the aqueous compositions of CE1, CE2 and IE1 to IE6 had a concentration of Snomax^{®} (i.e. INP-carrying bacteria/bacterial fragments) of 1 mg/ml. By dilution, the Snomax^{®} concentrations of the aqueous compositions of CE1, CE2 and IE1 to IE6 were stepwise reduced as indicated below in Tables 3 to 8. The phosphate buffer concentration in the aqueous compositions of CE2 and IE1 to IE6 and the concentration of the polyfunctional compound in the aqueous compositions of IE1 to IE6 (0.5 wt% for PVA, 1 wt% for all other polyfunctional compounds) remained unchanged.

For each Snomax^{®} concentration, the average ice nucleation temperature T₅₀ (which is the temperature at which 50% of the water droplets were frozen) was determined for the aqueous compositions of CE1, CE2 and IE1 to IE6. The higher the average ice nucleation temperature T₅₀, the higher is the ice nucleation efficiency of the composition.

The average ice nucleation temperature T₅₀ of an aqueous composition was determined as follows: 96 drops (each drop: 3 µl) of the aqueous composition were deposited on a substrate and cooled at a cooling rate of 1 °C/min. For each drop, the ice nucleation temperature was detected. T₅₀ corresponds to the temperature at which 50% of the drops were frozen.

The components of the aqueous compositions used in CE1, CE2 and IE1 to IE6 are listed in Table 2.

**Table 2: Components of the aqueous compositions of CE1, CE2 and IE1 to IE6**

| | Components of the aqueous composition |
|---|---|
| CE1 | (i) INP-carrying bacteria/bacterial fragments (Snomax^{®}) at different concentrations due to stepwise dilution |
| CE2 | (i) INP-carrying bacteria/bacterial fragments (Snomax^{®}) at different concentrations due to stepwise dilution |
| | (ii) Phosphate buffer (150 mM) |
| IE1 | (i) INP-carrying bacteria/bacterial fragments (Snomax^{®}) at different concentrations due to stepwise dilution |
| | (ii) Phosphate buffer (150 mM) |
| | (iii) Glycerol as a polyfunctional compound (1 wt%) |
| IE2 | (i) INP-carrying bacteria/bacterial fragments (Snomax^{®}) at different concentrations due to stepwise dilution |
| | (ii) Phosphate buffer (150 mM) |
| | (iii) Ethylene glycol as a polyfunctional compound (1 wt%) |
| IE3 | (i) INP-carrying bacteria/bacterial fragments (Snomax^{®}) at different concentrations due to stepwise dilution |
| | (ii) Phosphate buffer (150 mM) |
| | (iii) Polyethylene glycol as a polyfunctional compound (1 wt%) |
| IE4 | (i) INP-carrying bacteria/bacterial fragments (Snomax^{®}) at different concentrations due to stepwise dilution |
| | (ii) Phosphate buffer (150 mM) |
| | (iii) Polyethylene glycol polypropylene glycol block polymer as a polyfunctional compound (1 wt%) |
| IE5 | (i) INP-carrying bacteria/bacterial fragments (Snomax^{®}) at different concentrations due to stepwise dilution |
| | (ii) Phosphate buffer (150 mM) |
| | (iii) Polyvinyl alcohol as a polyfunctional compound (0.5 wt%) |
| IE6 | (i) INP-carrying bacteria/bacterial fragments (Snomax^{®}) at different concentrations due to stepwise dilution |
| | (ii) Phosphate buffer (150 mM) |
| | (iii) Alginate as a polyfunctional compound (1 wt%) |

The average ice nucleation temperature T₅₀ of the aqueous compositions of CE1, CE2 and IE1 to IE6 at different concentrations of the INP-carrying bacteria/bacterial fragments is listed below in Tables 3 to 8.

### Comparison between the aqueous compositions of IE1 and CE1 to CE2

As already mentioned above, the aqueous composition of CE2 differs from the aqueous composition of CE1 in that it comprises, in addition to the INP-carrying bacteria/bacterial fragments, a phosphate buffer, and the aqueous composition of IE1 differs from the aqueous composition of CE2 in that it comprises, in addition to the INP-carrying bacteria/bacterial fragments and the phosphate buffer, glycerol as a polyfunctional compound.

**Table 3: Average ice nucleation temperature T₅₀ of the aqueous compositions of CE1, CE2 and IE1 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE1 |
| | 1 | -2.96 | -3.28 | -3.29 |
| | 0.1 | -3.08 | -3.44 | -3.47 |
| | 1 × 10⁻² | -3.48 | -3.66 | -3.71 |
| | 1 × 10⁻³ | -4.51 | -4.02 | -3.96 |
| | 1 × 10⁻⁴ | -7.04 | -4.84 | -4.52 |
| | 1 × 10⁻⁵ | -7.75 | -6.39 | -5.57 |
| | 1 × 10⁻⁶ | -8.99 | -7.98 | -7.87 |

At concentrations of the INP-carrying bacteria/bacterial fragments of above 5 × 10⁻³ mg/ml, the aqueous compositions of CE1 and CE2 show a higher average ice nucleation temperature T₅₀ and therefore higher ice nucleation activity than the aqueous composition of IE1. However, it has surprisingly turned out that the the aqueous composition of IE1 shows a higher average ice nucleation temperature T₅₀ and therefore higher ice nucleation activity than the aqueous composition of CE1 and CE2 if the concentrations of the INP-carrying bacteria/bacterial fragments is reduced to less than 5 × 10⁻³ mg/ml.

Accordingly, due to the presence of the buffer and glycerol as a polyfunctional compound, the aqueous composition of IE1 has still a surprisingly high ice nucleation activity even if the INP-carrying bacteria/bacterial fragments are present at a very low concentration (0.005 mg/ml or less).

### Comparison between the aqueous compositions of IE2 and CE1 to CE2

As already mentioned above, the aqueous composition of CE2 differs from the aqueous composition of CE1 in that it comprises, in addition to the INP-carrying bacteria/bacterial fragments, a phosphate buffer; and the aqueous composition of IE2 differs from the aqueous composition of CE2 in that it comprises, in addition to the INP-carrying bacteria/bacterial fragments and the phosphate buffer, ethylene glycol as a polyfunctional compound.

**Table 4: Average ice nucleation temperature T₅₀ of the aqueous compositions of CE1, CE2 and IE2 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE2 |
| | 1 | -2.96 | -3.28 | -3.77 |
| | 0.1 | -3.08 | -3.44 | -3.79 |
| | 1 × 10⁻² | -3.48 | -3.66 | -3.97 |
| | 1 × 10⁻⁴ | -7.04 | -4.84 | -4.53 |
| | 1 × 10⁻⁵ | -7.75 | -6.39 | -5.29 |

At concentrations of the INP-carrying bacteria/bacterial fragments of above 5 × 10⁻³ mg/ml, the aqueous compositions of CE1 and CE2 show a higher average ice nucleation temperature T₅₀ and therefore higher ice nucleation activity than the aqueous composition of IE2. However, it has surprisingly turned out that the the aqueous composition of IE2 shows a higher average ice nucleation temperature T₅₀ and therefore higher ice nucleation activity than the aqueous composition of CE1 and CE2 if the concentrations of the INP-carrying bacteria/bacterial fragments is reduced to 1 × 10⁻⁴ mg/ml and 1 × 10⁻⁵ mg/ml (i.e. less than 5 × 10⁻³ mg/ml).

Accordingly, due to the presence of the buffer and ethylene glycol as a polyfunctional compound, the aqueous composition of IE2 has still a surprisingly high ice nucleation activity even if the INP-carrying bacteria/bacterial fragments are present at a very low concentration (0.005 mg/ml or less).

### Comparison between the aqueous compositions of IE3 and CE1 to CE2

As already mentioned above, the aqueous composition of CE2 differs from the aqueous composition of CE1 in that it comprises, in addition to the INP-carrying bacteria/bacterial fragments, a phosphate buffer; and the aqueous composition of IE3 differs from the aqueous composition of CE2 in that it comprises, in addition to the INP-carrying bacteria/bacterial fragments and the phosphate buffer, polyethylene glycol as a polyfunctional compound.

**Table 5: Average ice nucleation temperature T₅₀ of the aqueous compositions of CE1, CE2 and IE3 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE3 |
| | 1 | -2.96 | -3.28 | -2.91 |
| | 0.1 | -3.08 | -3.44 | -3.13 |
| | 1 × 10⁻² | -3.48 | -3.66 | -3.39 |
| | 1 × 10⁻³ | -4.51 | -4.02 | -3.87 |
| | 1 × 10⁻⁴ | -7.04 | -4.84 | -4.71 |
| | 1 × 10⁻⁵ | -7.75 | -6.39 | -6.04 |
| | 1 × 10⁻⁶ | -8.99 | -7.98 | -7.56 |

At concentrations of the INP-carrying bacteria/bacterial fragments of less than 5 × 10⁻³ mg/ml, the aqueous composition of IE3 shows a higher average ice nucleation temperature T₅₀ and therefore higher ice nucleation activity than the aqueous composition of CE1 and CE2.

Accordingly, due to the presence of the buffer and polyethylene glycol as a polyfunctional compound, the aqueous composition of IE3 has still a surprisingly high ice nucleation activity even if the INP-carrying bacteria/bacterial fragments are present at a very low concentration (0.005 mg/ml or less).

### Comparison between the aqueous compositions of IE4 and CE1 to CE2

As already mentioned above, the aqueous composition of CE2 differs from the aqueous composition of CE1 in that it comprises, in addition to the INP-carrying bacteria/bacterial fragments, a phosphate buffer; and the aqueous composition of IE4 differs from the aqueous composition of CE2 in that it comprises, in addition to the INP-carrying bacteria/bacterial fragments and the phosphate buffer, a polyethylene glycol polypropylene glycol block polymer as a polyfunctional compound.

**Table 6: Average ice nucleation temperature T₅₀ of the aqueous compositions of CE1, CE2 and IE4 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE4 |
| | 1 | -2.96 | -3.28 | -3.05 |
| | 0.1 | -3.08 | -3.44 | -3.19 |
| | 1 × 10⁻² | -3.48 | -3.66 | -3.43 |
| | 1 × 10⁻³ | -4.51 | -4.02 | -3.69 |
| | 1 × 10⁻⁴ | -7.04 | -4.84 | -3.99 |
| | 1 × 10⁻⁵ | -7.75 | -6.39 | -5.42 |
| | 1 × 10⁻⁶ | -8.99 | -7.98 | -7.59 |

At concentrations of the INP-carrying bacteria/bacterial fragments of less than 5 × 10⁻³ mg/ml, the aqueous composition of IE4 shows a higher average ice nucleation temperature T₅₀ and therefore higher ice nucleation activity than the aqueous composition of CE1 and CE2.

Accordingly, due to the presence of the buffer and the polyethylene glycol polypropylene glycol block polymer as a polyfunctional compound, the aqueous composition of IE4 has still a surprisingly high ice nucleation activity even if the INP-carrying bacteria/bacterial fragments are present at a very low concentration (0.005 mg/ml or less).

### Comparison between the aqueous compositions of IE5 and CE1 to CE2

As already mentioned above, the aqueous composition of CE2 differs from the aqueous composition of CE1 in that it comprises, in addition to the INP-carrying bacteria/bacterial fragments, a phosphate buffer; and the aqueous composition of IE5 differs from the aqueous composition of CE2 in that it comprises, in addition to the INP-carrying bacteria/bacterial fragments and the phosphate buffer, polyvinyl alcohol as a polyfunctional compound.

**Table 7: Average ice nucleation temperature T₅₀ of the aqueous compositions of CE1, CE2 and IE5 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE5 |
| | 1 | -2.96 | -3.28 | -3.29 |
| | 0.1 | -3.08 | -3.44 | -3.46 |
| | 1 × 10⁻² | -3.48 | -3.66 | -3.66 |
| | 1 × 10⁻³ | -4.51 | -4.02 | -3.88 |
| | 1 × 10⁻⁴ | -7.04 | -4.84 | -4.37 |
| | 1 × 10⁻⁵ | -7.75 | -6.39 | -5.51 |

At concentrations of the INP-carrying bacteria/bacterial fragments of less than 5 × 10⁻³ mg/ml, the aqueous composition of IE5 shows a higher average ice nucleation temperature T₅₀ and therefore higher ice nucleation activity than the aqueous composition of CE1 and CE2.

Accordingly, due to the presence of the buffer and polyvinyl alcohol as a polyfunctional compound, the aqueous composition of IE5 has still a surprisingly high ice nucleation activity even if the INP-carrying bacteria/bacterial fragments are present at a very low concentration (0.005 mg/ml or less).

### Comparison between the aqueous compositions of IE6 and CE1 to CE2

As already mentioned above, the aqueous composition of CE2 differs from the aqueous composition of CE1 in that it comprises, in addition to the INP-carrying bacteria/bacterial fragments, a phosphate buffer; and the aqueous composition of IE6 differs from the aqueous composition of CE2 in that it comprises, in addition to the INP-carrying bacteria/bacterial fragments and the phosphate buffer, sodium alginate as a polyfunctional compound.

**Table 8: Average ice nucleation temperature T₅₀ of the aqueous compositions of CE1, CE2 and IE6 at different concentrations of the INP-carrying bacteria/bacterial fragments**

| Concentration of the INP-carrying bacteria/bacterial fragments in the aqueous composition [mg/ml] | | T₅₀ [°C] | | |
|---|---|---|---|---|
| | | Aqueous composition of CE1 | Aqueous Composition of CE2 | Aqueous composition of IE6 |
| | 1 | -2.96 | -3.28 | -3.46 |
| | 0.1 | -3.08 | -3.44 | -3.66 |
| | 1 × 10⁻² | -3.48 | -3.66 | -3.84 |
| | 1 × 10⁻⁴ | -7.04 | -4.84 | -4.78 |
| | 1 × 10⁻⁵ | -7.75 | -6.39 | -5.40 |
| | 1 × 10⁻⁶ | -8.99 | -7.98 | -7.49 |

At concentrations of the INP-carrying bacteria/bacterial fragments of above 5 × 10⁻³ mg/ml, the aqueous compositions of CE1 and CE2 show a higher average ice nucleation temperature T₅₀ and therefore higher ice nucleation activity than the aqueous composition of IE6. However, it has surprisingly turned out that the the aqueous composition of IE6 shows a higher average ice nucleation temperature T₅₀ and therefore higher ice nucleation activity than the aqueous composition of CE1 and CE2 if the concentration of the INP-carrying bacteria/bacterial fragments is reduced to less than 5 × 10⁻³ mg/ml.

Accordingly, due to the presence of the buffer and sodium alginate as a polyfunctional compound, the aqueous composition of IE6 has still a surprisingly high ice nucleation activity even if the INP-carrying bacteria/bacterial fragments are present at a very low concentration (0.005 mg/ml or less).

## Claims

1. An aqueous composition comprising
(i) 0.005 mg/ml or less of bacteria, bacterial ghosts or fragments thereof which carry an ice nucleation protein,
(ii) a buffer,
(iii) a polyfunctional compound comprising two or more functional groups,
wherein the functional groups are selected from a hydroxy group, an ether group, a carboxylic acid or carboxylate group, a carboxylic acid ester group, an amine group and an amide group,
wherein the aqueous composition has a pH of from 5 to 8.

2. The aqueous composition according to claim 1, wherein the bacteria are selected from *Pseudomonas syringae*, *Pseudomonas fluorescens*, *Xanthomonas campestris*, *Pantoea ananas*, *Pantoea agglomerans* and *Enterobacter agglomerans*; and/or the ice nucleation protein is selected from InaZ, InaX, InaA, InaV, InaU, InaK, InaW and IceE.

3. The aqueous composition according to claim 1 or 2, wherein the buffer is a phosphate buffer which comprises H₂PO4⁻ and/or HPO4²⁻.

4. The aqueous composition according to one of the preceding claims, wherein the polyfunctional compound comprises at least two hydroxy groups and optionally one or more functional groups which are selected from an ether group, a carboxylic acid or carboxylate group and a carboxylic acid ester group

5. The aqueous composition according to one of the preceding claims, wherein the polyfunctional compound is a monoalkylene glycol, a polyalkylene glycol, glycerol, a polyvinyl alcohol or a polysaccharide.

6. The aqueous composition according to claim 5, wherein the monoalkylene glycol is ethylene glycol or propylene glycol.

7. The aqueous composition according to claim 5, wherein the polyalkylene glycol is a homo- or copolymer, preferably an ethylene glycol homo- or copolymer or a propylene glycol homo- or copolymer.

8. The aqueous composition according to claim 7, wherein the polyalkylene glycol copolymer is a block polymer comprising at least one polyethylene glycol block and at least one polypropylene glycol block.

9. The aqueous composition according to claim 5, wherein the polysaccharide is alginic acid or a salt thereof.

10. The aqueous composition according to one of the preceding claims, comprising one or more salts at a concentration of 0.001 mol/I to 2 mol/I, wherein the one or more salts are selected from one or more alkali metal salts, one or more earth alkali metal salts, one or more aluminium salts, one or more iron salts, and one or more manganese salts.

11. Use of the aqueous composition according to one of the claims 1 to 10 as an ice nucleating agent.

12. Use according to claim 11 for snowmaking, freezing of foodstuff or foodstuff precursors, ex-vivo cryopreservation of biological samples, or cloud seeding.

13. A process of forming artificial snow wherein the aqueous composition according to one of the claims 1 to 10 is sprayed into the air by a snowmaking machine.

14. A process of cloud seeding wherein the aqueous composition according to one of the claims 1 to 10 is sprayed into an atmospheric cloud.

15. A process of freezing foodstuff or a foodstuff precursor wherein the aqueous composition according to one of the claims 1 to 10 is in thermal contact with the foodstuff or foodstuff precursor and cooled to a temperature at which ice crystals are formed.

16. A process of ex-vivo cryopreservation of a biological sample wherein the aqueous composition according to one of the claims 1 to 10 is in thermal contact with the biological sample outside of the human or animal body and is cooled to a temperature at which ice crystals are formed.
